(19) Europäisches Patentamt
European Patent Office
Office européen des brevets

(11) Veröffentlichungsnummer: **0 399 310 A1**

(12) **EUROPÄISCHE PATENTANMELDUNG**

(21) Anmeldenummer: **90108892.2**

(51) Int. Cl.[5]: **C07D 253/06, A01N 43/707**

(22) Anmeldetag: **11.05.90**

(30) Priorität: **25.05.89 DE 3917044**

(43) Veröffentlichungstag der Anmeldung:
**28.11.90 Patentblatt 90/48**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Kranz, Eckart, Dr.**
**Am Acker 9**
**D-5600 Wuppertal 1(DE)**
Erfinder: **Reubke, Karl-Julius, Dr.**
**Rybniker Strasse 10**
**D-5000 Köln 80(DE)**
Erfinder: **Lürssen, Klaus, Dr.**
**August-Kierspel-Strasse 145**
**D-5060 Bergisch Gladbach 2(DE)**
Erfinder: **Santel, Hans-Joachim, Dr.**
**Gruenstrasse 9a**
**D-5090 Leverkusen(DE)**
Erfinder: **Schmidt, Robert R., Dr.**
**Im Waldwinkel 110**
**D-5060 Bergisch Gladbach 2(DE)**
Erfinder: **Krauskopf, Birgit, Dr.**
**Kicke 19**
**D-5060 Bergisch Gladbach 1(DE)**

(54) **6-(Pent-3-yl)-1,2,4-triazin-5 (4H)-one, Verfahren zu ihrer Herstellung und ihre Verwendung als Herbizide und Pflanzenwachstumsregulatoren.**

(57) Die Erfindung betrifft neue 6-(Pent-3-yl)-1,2,4-triazin-5(4H)-one der Formel (I),

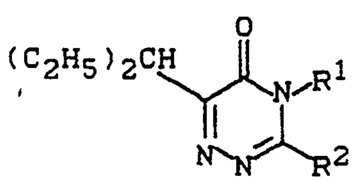

(I)

in welcher
$R^1$ für Amino und
$R^2$ für Alkylthio mit mehr als 1 Kohlenstoffatom, Alkylamino oder Dialkylamino steht,
oder
$R^1$ für Methylamino und
$R^2$ für Alkylthio steht,
Verfahren zu ihrer Herstellung und ihre Verwendung als Herbizide und Pflanzenwachstumsregulatoren.

EP 0 399 310 A1

## 6-(Pent-3-yl)-1,2,4-triazin-5(4H)-one, Verfahren zu ihrer Herstellung und ihre Verwendung als Herbizide und Pflanzenwachstumsregulatoren

Die vorliegende Erfindung betrifft neue 6-(Pent-3-yl)-1,2,4-triazin-5(4H)-one, mehrere Verfahren zu ihrer Herstellung und ihre Verwendung als Herbizide und Pflanzenwachstumsregulatoren.

Es ist bereits bekannt geworden, daß bestimmte 6-sec-Pentyl-1,2,4-triazin-5(4H)-one, wie beispielsweise 4-Amino-3-methylthio-6-(pent-3-yl)-1,2,4-triazin-5(4H)-on, herbizide Eigenschaften besitzen (vgl. DE-OS 33 39 859).

Ferner sind verschiedene substiutierte 6-Alkyl-3,4-diamino-1,2,4-triazin-5-(4H)-one und deren herbizide und insektizide Eigenschaften beschrieben worden (vgl. EP-A 0 150 677).

Außerdem ist bereits bekannt, daß symmetrische Triazin-Derivate, wie beispielsweise 2-Chlor-4-ethylamino-6-iso propylamino-1,3,5-triazin (Atrazin) herbizide Eigenschaften aufweisen (vgl. R. Wegler, Chemie der Pflanzenschutz- und Schädlingsbekämpfungsmittel, Band 2, S. 368, Springer-Verlag, 1970).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Es wurden die neuen 6-(Pent-3-yl)-1,2,4-triazin-5(4H)-one der Formel (I)

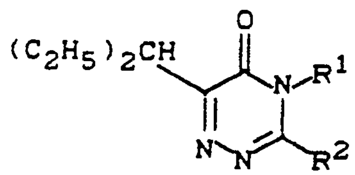

(I)

in welcher
$R^1$ für Amino und
$R^2$ für Alkylthio mit mehr als 1 Kohlenstoffatom, Alkylamino oder Dialkylamino steht,
oder
$R^1$ für Methylamino und
$R^2$ für Alkylthio steht,
gefunden.

Weiterhin wurde gefunden, daß man die neuen 6-(Pent-3-yl)-1,2,4-triazin-5(4H)-one der Formel (I) erhält, wenn man

(a) 4-Amino-3-mercapto-6-(pent-3-yl)-1,2,4-triazin-5(4H)-on der Formel (II)

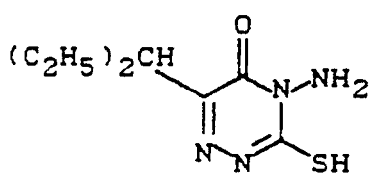

(II)

in alkalischer Lösung mit einem Alkylhalogenid, vorzugsweise Alkyliodid oder -bromid, umsetzt und gegebenenfalls

(b) die nach Verfahren (a) erhaltenen 3-Alkylthio-4-amino-6-(pent-3-yl)-1,2,4-triazin-5(4H)-one der Formel (Ia)

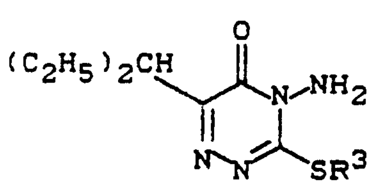

(Ia)

in welcher
$R^3$ für Alkyl, vorzugsweise mit 1 bis 4 Kohlenstoffatomen, steht,

mit einem Methylierungsmittel in Gegenwart eines Phasentransferkatalysators in einem Zweisphasensystem umsetzt, oder

(c) die nach Verfahren (a) erhaltenen 3-Alkylthio-4-amino-6-(pent-3-yl)-1,2,4-triazin-5(4H)-one der Formel (Ia)

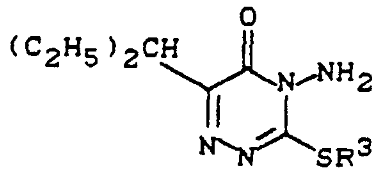

(Ia)

in welcher

$R^3$ für Alkyl, vorzugsweise mit 1 bis 4 Kohlenstoffatomen, steht,

mit Aminen der Formel (III)

$HNR^4R^5$ (III)

in welcher

$R^4$ für Wasserstoff oder Alkyl, vorzugsweise mit 1 bis 4 Kohlenstoffatomen, steht und

$R^5$ für Alkyl, vorzugsweise mit 1 bis 4 Kohlenstoffatomen, steht,

gegebenenfalls in Gegenwart eines Verdünnungsmittels und gegebenenfalls in Gegenwart einer niederen aliphatischen Carbonsäure umsetzt.

Schließlich wurde gefunden, daß die neuen 6-(Pent-3-yl)-1,2,4-triazin-5(4H)-one der Formel (I) herbizide, insbesondere selektiv-herbizide und pflanzenwachstumsregulierende Eigenschaften besitzen.

Überraschenderweise zeigen die erfindungsgemäßen 6-(Pent-3-yl)-1,2,4-triazin-5(4H)-one der Formel (I) eine bessere herbizide Wirksamkeit gegenüber wichtigen Problemunkräutern bei gleich guter Verträglichkeit gegenüber wichtigen Kulturpflanzen, wie insbesondere Weizen, Gerste, Mais, Soja und Baumwolle, beispielsweise im Vergleich zu 2-Chlor-4-ethylamino-6-isopropylamino-1,3,5-triazin, welches chemisch und wirkungsmäßig eine naheliegende Verbindung ist.

Darüberhinaus zeigen die erfindungsgemäßen Verbindungen der Formel (I) zusätzlich eine pflanzenwachstumsregulierende Wirkung.

Die erfindungsgemäßen 6-(Pent-3-yl)-1,2,4-triazin-5(4H)-one sind durch die Formel (I) allgemein definiert. In dieser Formel stehen vorzugsweise

$R^1$ für Amino und

$R^2$ für geradkettiges oder verzweigtes Alkylthio mit 2 bis 4 Kohlenstoffatomen, für geradkettiges oder verzweigtes Alkylamino mit 1 bis 4 Kohlenstoffatomen sowie für geradkettiges oder verzweigtes Dialkylamino mit 1 bis 4 Kohlenstoffatomen in jedem Alkylteil;

oder

$R^1$ für Methylamino und

$R^2$ für geradkettiges oder verzweigtes Alkylthio mit 1 bis 4 Kohlenstoffatomen.

Besonders bevorzugt sind diejenigen Verbindungen der Formel (I), in denen

$R^1$ für Amino und

$R^2$ für Ethyl- oder Propylthio; für Methyl-, Ethyl-, Propyl- oder Butylamino; sowie für Dimethyl-, Diethyl- oder Ethylmethylamino stehen;

oder

$R^1$ für Methylamino und

$R^2$ für Methyl-, Ethyl- oder Propylthio stehen.

Verwendet man beispielsweise 4-Amino-3-mercapto-6-(pent-3-yl)-1,2,4-triazin-5(4H)-on und Ethyliodid als Ausgangsstoffe, so läßt sich der Reaktionsablauf des erfindungsgemäßen Verfahrens (a) durch das folgende Formelschema wiedergeben:

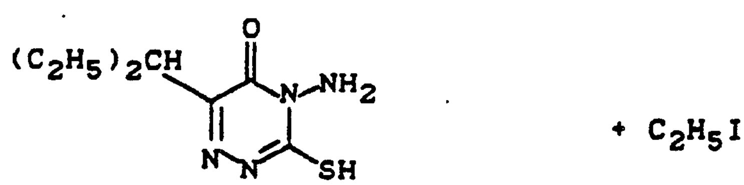

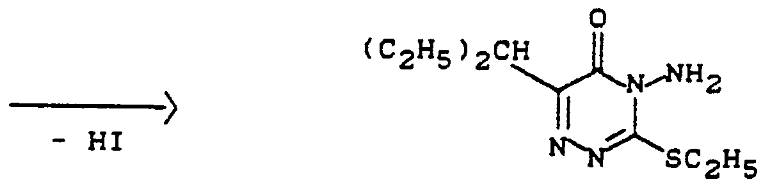


Verwendet man beispielsweise 4-Amino-3-methylthio-6-(pent-3-yl)-1,2,4-triazin-5(4H)-on und Methyliodid als Ausgangsstoffe, Tetrabutylammoniumbromid als Phasentransferkatalysator und wässrige Natronlauge-Toluol als Zweiphasensystem, so läßt sich der Reaktionsablauf des erfindungsgemäßen Verfahrens (b) durch das folgende Formelschema wiedergeben:

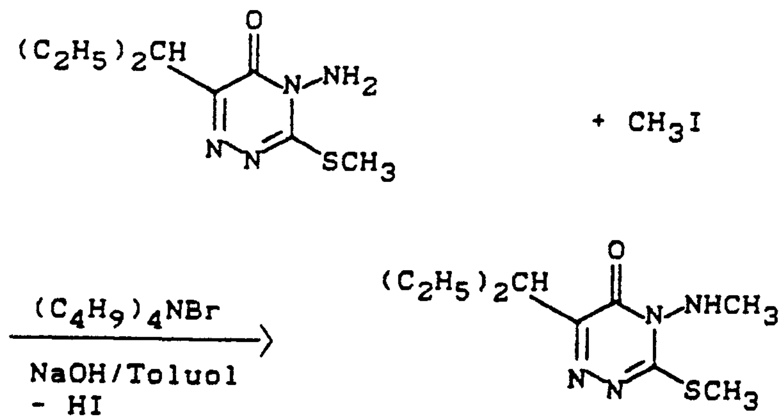


Verwendet man beispielsweise 4-Amino-3-methylthio-6-(pent-3-yl)-1,2,4-triazin-5(4H)-on und Methylamin als Ausgangsstoffe, so läßt sich der Reaktionsablauf des erfindungsgemäßen Verfahrens (c) durch das folgende Formelschema wiedergeben:

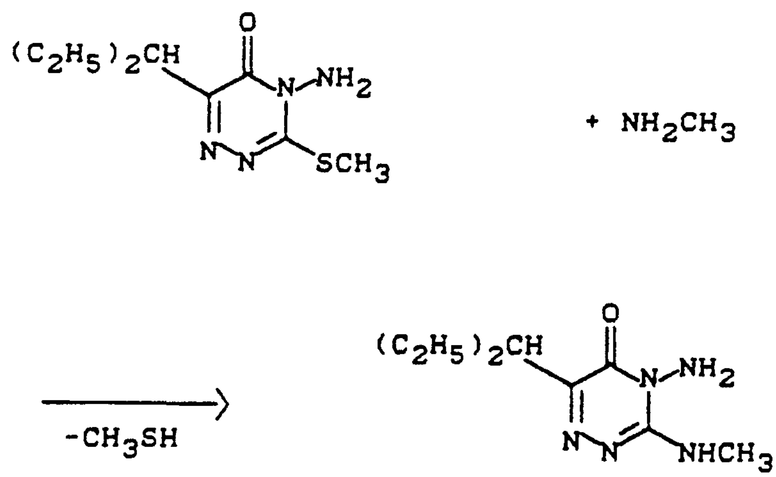


Das zur Durchführung des erfindungsgemäßen Verfahrens (a) als Ausgangsstoff benötigte 4-Amino-3-mercapto-6-(pent-3-yl)-1,2,4-triazin-5(4H)-on der Formel (II) und dessen Herstellung sind bereits beschrieben (vergleiche DE-OS 33 39 859 sowie die Herstellungsbeispiele).

Als Methylierungsmittel kommen für das erfindungsgemäße Verfahren (b) vorzugsweise Methyliodid, Methylbromid oder Dimethylsulfat infrage.

Die zur Durchführung des erfindungsgemäßen Verfahrens (c) als Ausgangsstoffe benötigten Amine der Formel (III) sind allgemein bekannte Verbindungen der organischen Chemie.

Das erfindungsgemäße Verfahren (a) wird in Gegenwart einer Base durchgeführt. Hierbei werden vorzugsweise Alkalihydroxide, wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung des erfindungsgemäßen Verfahrens (a) in einem größeren Bereich variiert werden. Im allgemeinen arbeitet man zwischen 0° C und 100° C, vorzugsweise zwischen 0° C und 50° C.

Bei der Durchführung des erfindungsgemäßen Verfahrens (a) setzt man auf 1 Mol an Verbindung der Formel (II) vorzugsweise 1 bis 1,5 Mol Alkylierungsmittel ein. Die Isolierung der Zwischenprodukte bzw. Endprodukte der Formel (Ia) erfolgt in üblicher Weise.

Das erfindungsgemäße Verfahren (b) wird in einem Zweiphasensystem durchgeführt, vorzugsweise mit einem organischen, mit Wasser nicht mischbaren Lösungsmittel sowie einer wässrigen Lösung einer starken Base und eines Phasentransferkatalysators.

Als organische Lösungsmittel kommen vorzugsweise in Betracht: aliphatische und aromatische Kohlenwasserstoffe, wie beispielsweise Benzol, Toluol, Xylol, Petrolether und Cyclohexan; halogenierte Kohlenwasserstoffe, wie Chlorbenzol, Methylenchlorid, Chloroform und Tetrachlorkohlenstoff; Ether, wie Diethylether und Diisopropylether; sowie deren Gemische.

Als starke Basen kommen vorzugsweise in Betracht: Alkalimetallhydroxide, wie Natrium- oder Kaliumhydroxid, sowie Alkali- oder Erdalkalicarbonate.

Als Phasentransferkatalysatoren kommen vorzugsweise in Betracht: quarternäre Ammoniumsalze oder -hyroxide sowie Phosphoniumsalze. Als Beispiel seien genannt: Tetra-n-butylammoniumbromid, Benzyltriethylammoniumchlorid, Tetra-n-butylammoniumhydroxid, Benzyltrimethylammoniumchlorid, Tributylhexadecylphosphoniumbromid, Ethyltriphenylphosphoniumbromid, Tetraphenylphosphoniumchlorid, Benzyltriphenylphosphoniumiodid oder Tetrabutylphosphoniumchlorid.

Die Reaktionstemperaturen können bei der Durchführung des erfindungsgemäßen Verfahrens (b) in einem größeren Bereich variiert werden. Im allgemeinen arbeitet man zwischen 10° C und 50° C, vorzugsweise zwischen 20° C und 30° C.

Bei der Durchführung des erfindungsgemäßen Verfahrens (b) setzt man auf 1 Mol an Verbindung der Formel (Ia) vorzugsweise 1 bis 3 Mol Methylierungsmittel ein. Die Isolierung der Zwischenprodukte bzw. Endprodukte der Formel (Ib) erfolgt in üblicher Weise.

Für das erfindungsgemäße Verfahren (c) kommen als Verdünnungsmittel alle inerten organischen Lösungsmittel infrage. Hierzu gehören Kohlenwasserstoffe, wie Toluol, Xylol; chlorierte aromatische Kohlenwasserstoffe, wie Chlorbenzol, 1,2-Dichlorbenzol, 1,2,4-Trichlorbenzol; Ether, wie Tetrahydrofuran, Dioxan; Alkohole, wie Methanol, Ethanol, Propanol, Isopropanol; Amide, wie N,N-Dimethylformamid, Tetramethylharnstoff oder Sulfoxide, wie Dimethylsulfoxid. Vorzugsweise wird für die Umsetzung Isopropanol verwendet.

Die Reaktionstemperaturen können bei der Durchführung des erfindungsgemäßen Verfahrens (c) in einem größeren Bereich variiert werden. Im allgemeinen arbeitet man bei Temperaturen zwischen 20° C und 170° C, vorzugsweise bei Temperaturen zwischen 60° C und 90° C.

Eine besonders vorteilhafte Ausführungsform des erfindungsgemäßen Verfahrens (c) besteht darin, daß man in Gegenwart mindestens der äquimolaren Menge einer niederen aliphatischen Carbonsäure arbeitet. Vorzugsweise wird hierfür Essigsäure verwendet. Dieses Verfahren gestattet es mit relativ geringem Amin-Überschuß auszukom men. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfonsäure.

Bei der Durchführung des erfindungsgemäßen Verfahrens (c) setzt man auf 1 Mol des 3-Alkylthiotriazinons der Formel (Ia) zweckmäßigerweise 1 bis 3 Mol einer niederen aliphatischen Carbonsäure und gegebenenfalls 0,01 bis 0,05 Mol einer organischen Sulfonsäure sowie 1 bis 7 Mol Amin der Formel (III) ein, erhitzt bis zum Ende der Mercaptan-Abspaltung und arbeitet anschließend in üblicher Art und Weise auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Bete, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung mono- und dikotyler Unkräuter in mono- und dikotoylen Kulturen, insbesondere in Weizen, Gerste, Mais, Soja und Baumwolle, einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen des Wirkstoffs mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N′-(3-trifluormethylphenyl)-harnstoff (FLUOMETURON); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)oxyl-phenoxy}-propansäure bzw. deren Ethylester (HALOXYFOP); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N′-(4-isopropylphenyl)-harnstoff (ISOPROTURON); (2-Ethoxy-1-methyl-2- oxo-ethyl)-5-[2-chlor-4-(trifluormethyl)-phenoxy)-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)amino)-carbonyl]-amino]-sulfonyl}-benzoesäure oder deren Methylester (METSULFURON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); O-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]N,N-diethyl-thiocarbamat (THIOBENCARB); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN) sind möglich.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können bei der Anwendung als Wachstumsregulatoren in den Formulierungen ebenfalls in Mischung mit anderen bekannten Wirkstoffen vorliegen, wie Fungizide, Insektizide, Akarizide und Herbizide sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z. B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemässen Wirkstoffe gehen aus den nachfolgenden Beispielen hervor.

## Herstellungsbeispiele

### Beispiel 1

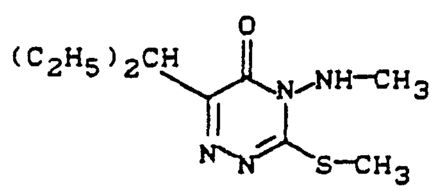

(Verfahren b)

4,2 g (0,018 Mol) 4-Amino-3-methylthio-6-(pent-3-yl)-1,2,4-triazin-5(4H)-on werden bei Raumtemperatur in eine Mischung von 7,5 ml 45 %iger Natronlauge und 7,5 ml Toluol eingetragen. Bei Raumtemperatur gibt man anschließend 7,1 g (0,05 Mol) Methyliodid und 0,6 g Tetrabutylammoniumbromid auf einmal zu. Bei intensivem Rühren steigt die Temperatur von 21 °C auf 31 °C an. Man läßt das Reaktionsgemisch 1 Stunde nachrühren, trennt danach die organische Oberphase im Scheidetrichter ab und verrührt diese in 100 ml Wasser. Nach Extraktion mit Methylenchlorid (dreimal je 70 ml) und anschließender Filtration über Kieselgel erhält man nach dem Einengen 3,8 g (87 % der Theorie) 4-Methylamino-3-methylthio-6-(pent-3-yl)-1,2,4-triazin-5(4H)-on als farbloses Öl mit dem Brechungsindex $n_D^{20}$ = 1,553.

### Herstellung des Ausgangsproduktes

(a)

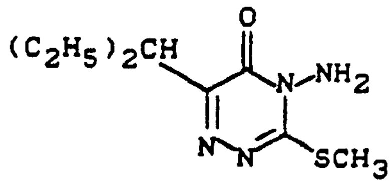

(Verfahren a)

50 g (0,23 Mol) 4-Amino-3-mercapto-6-(pent-3-yl)-1,2,4-triazin-5(4H)-on werden in 260 ml 1N Natronlauge gelöst und vom unlöslichen Rückstand abfiltriert. Das Filtrat wird bei Raumtemperatur mit 36,9 g (0,26 Mol) Methyliodid versetzt und 15 Stunden bei dieser Temperatur verrührt. Der ausgefallene Feststoff wird abgesaugt, mehrfach mit Wasser gewaschen und im Vakuum bei Raumtemperatur getrocknet.

Nach Filtration in Methylenchlorid über Kieselgel erhält man 41,5 g (79 % der Theorie) 4-Amino-3-methylthio-6-(pent-3-yl)-1,2,4-triazin-5(4H)-on vom Schmelzpunkt 50 °C bis 56 °C.

(b)

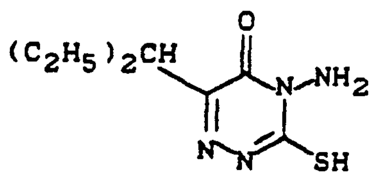

51,6 g (0,25 Mol) 3,3-Diethyl-brenztraubensäurenitril werden in 183,8 g einer 33 %igen Bromwasserstofflösung in Eisessig 1 Stunde bei 15 °C bis 20 °C gerührt.

Danach fügt man tropfenweise 4,5 g Wasser unter Rühren und Eiskühlung so zu, daß die Temperatur der Reaktionsmischung 20 °C nicht übersteigt. Man rührt 1 Stunde bei Raumtemperatur nach. Anschließend tropft man bei 15 °C bis 20 °C eine Lösung von 29,2 g (0,275 Mol) Thiocarbohydrazid in 275 ml 1N Salzsäure unter Kühlung zu. Nach beendeter Zugabe rührt man 15 Stunden bei Raumtemperatur nach. Das ausgefallene kristalline Produkt wird abgesaugt und mit Wasser neutral gewaschen.

Nach dem Trocknen bei 50 °C im Vakuum erhält man 40,1 g (75 % der Theorie) 4-Amino-3-mercapto-6-(pent-3-yl)-1,2,4-triazin-5(4H)-on vom Schmelzpunkt 162 °C bis 164 °C.

(c) $CH_3-CH_2-\underset{\displaystyle COCN}{\underset{|}{CH}}-CH_2-CH_3$

92,9 g (0,69 Mol) Pentan-3-carbonsäurechlorid werden auf 120 °C erwärmt. Bei dieser Temperatur tropft man innerhalb 2 Stunden 68,3 g (0,69 Mol) Trimethylsilylcyanid zu. Man läßt 6 Stunden bei einer Badtemperatur von 120 °C nachrühren, anschließend wird das entstandene Trimethylsilylchlorid bei Normaldruck abdestilliert. Das Reaktionsprodukt wird über eine 60 cm Spiegelvigreuxkolonne mit Rektifikationsaufsatz (Rücklauf 5 : 1) fraktioniert.

Man erhält 69,9 g (81 % der Theorie) 3,3-Diethylbrenztraubensäurenitril vom Siedepunkt 48 °C bis 56 °C/20 mbar.

<u>Beispiel 2</u>

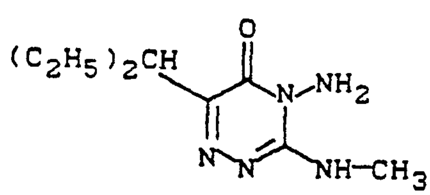

(Verfahren c)

In eine Lösung von 12 g (0,2 Mol) Eisessig in 150 ml Isopropanol werden bei 5 °C bis 10 °C 6,2 g (0,2 Mol) Monomethylamin eingeleitet. Diese Reaktionslösung wird anschließend mit 11,4 g (0,05 Mol) 4-Amino-3-methylthio-6-(pent-3-yl)-1,2,4-triazin-5(4H)-on versetzt. Man läßt auf Raumtemperatur erwärmen und erhitzt anschließend 24 Stunden unter Rückfluß. Danach wird das Reaktionsgemisch eingeengt und der ölige Rückstand in Wasser eingerührt. Man extrahiert mit Dichlormethan, wäscht mit Wasser, trocknet über Magnesiumsulfat und engt ein. Der Rückstand wird über Kieselgel-Filtration mit Essigester gereinigt.

Man erhält 6,8 g (64 % der Theorie) 4-Amino-3-methylamino-6-(pent-3-yl)-1,2,4-triazin-5(4H)-on vom Schmelzpunkt 119 °C bis 120 °C.

Beispiel 3

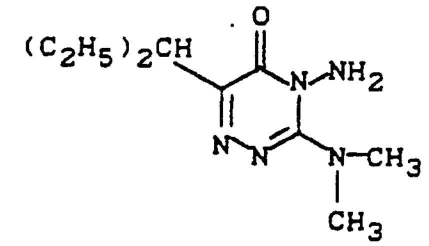

(Verfahren c)

In eine Lösung aus 12 g (0,2 Mol) Eisessig in 150 ml Isopropanol werden bei 5 °C bis 10 °C 9.0 g (0.2 Mol) Dimethylamin eingeleitet. Diese Reaktionslösung wird anschließend mit 11,4 g (0.05 Mol) 4-Amino-3-methylthio-6-(pent-3-yl)-1,2,4-triazin-5(4H)-on versetzt. Man läßt auf Raumtemperatur erwärmen und erhitzt anschließend 24 Stunden unter Rückfluß. Danach wird das Reaktionsgemisch eingeengt und der Rückstand in Dichlormethan aufgenommen. Man wäscht dreimal mit Wasser, trocknet über Magnesiumsulfat und engt ein. Der ölige Rückstand wird über eine Kieselgel-Säulenchromatografie mit dem Fließmittel Essigester gereinigt. Man erhält 8,1 g (72 % der Theorie) 4-Amino-3-dimethylamino-6-(pent-3-yl)-1,2,4-triazin-5(4H)-on mit dem Brechungsindex $n_D^{20}$ = 1.545.

In analoger Weise und entsprechend den erfindungsgemässen Verfahren werden die in der nachfolgenden Tabelle aufgeführten Verbindungen der Formel (I) erhalten:

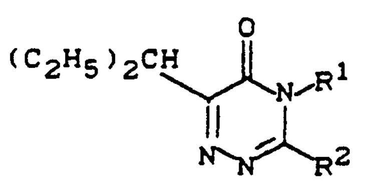

(I)

| Beispiel Nr. | $R^1$ | $R^2$ | Physikal. Konst. |
|---|---|---|---|
| 4 | $-NH_2$ | $-N(CH_3)C_2H_5$ | $n_D^{20}$ = 1,541 |

Anwendungsbeispiele

In den folgenden Anwendungsbeispielen wird die nachstehend aufgeführte Verbindung als Vergleichssubstanz eingesetzt:

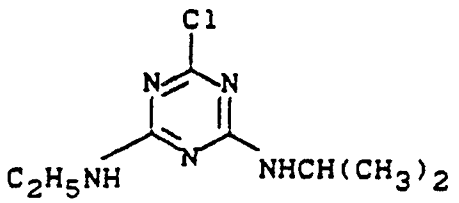

(A)
(Atrazin)

(bekannt aus R. Wegler, Chemie der Pflanzenschutz- und Schädlingsbekämpfungsmittel, Band 2, S. 368, Springer-Verlag, 1970)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z. B. die Verbindungen gemäß den Herstellungsbeispielen: 1, 3 und 4.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B die Verbindungen gemäß den Herstellungsbeispielen: 2, 3 und 4.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

Eine deutliche Überlegenheit im Vergleich zur unbehandelten Kontrolle zeigen in diesem Test beispielsweise die Verbindungen gemäß den Herstellungsbeispielen: 2 und 3.

## Ansprüche

1. 6-(Pent-3-yl)-1,2,4-triazin-5(4H)-one der Formel (I)

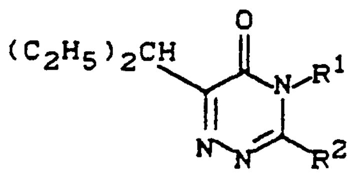

(I)

in welcher
$R^1$ für Amino und
$R^2$ für Alkylthio mit mehr als 1 Kohlenstoffatom, Alkylamino oder Dialkylamino steht,
oder
$R^1$ für Methylamino und
$R^2$ für Alkylthio steht.

2. Triazinon-Derivate der Formel (I) gemäß Anspruch 1, dadurch gekennzeichnet, daß darin
$R^1$ für Amino steht und
$R^2$ für geradkettiges oder verzweigtes Alkylthio mit 2 bis 4 Kohlenstoffatomen, für geradkettiges oder verzweigtes Alkylamino mit 1 bis 4 Kohlenstoffatomen sowie für geradkettiges oder verzweigtes Dialkylamino mit 1 bis 4 Kohlenstoffatomen in jedem Alkylteil steht;
oder
$R^1$ für Methylamino steht und
$R^2$ für geradkettiges oder verzweigtes Alkylthio mit 1 bis 4 Kohlenstoffatomen steht.

3. Triazinon-Derivate der Formel (I) gemäß Anspruch 1, dadurch gekennzeichnet, daß darin
$R^1$ für Amino steht und
$R^2$ für Ethyl- oder Propylthio; für Methyl-, Ethyl-, Propyl- oder Butylamino; sowie für Dimethyl-, Diethyl- oder Ethylmethylamino steht;
oder
$R^1$ für Methylamino steht und
$R^2$ für Methyl-, Ethyl- oder Propylthio steht.

4. a) 4-Methylamino-3-methylthio-6-(pent-3-yl)-1,2,4-triazin-5(4H)-on der Formel

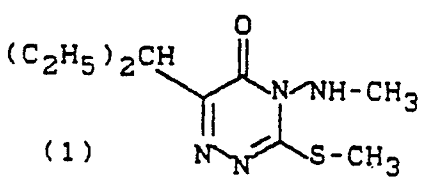

(1) ,

b) 4-Amino-3-methylamino-6-(pent-3-yl)-1,2,4-triazin-5(4H)-on der Formel

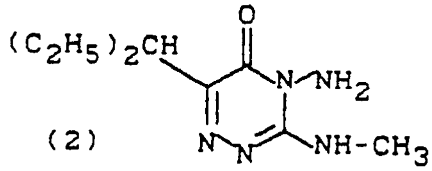

(2) ,

c) 4-Amino-3-dimethylamino-6-(pent-3-yl)-1,2,4-triazin-5(4H)-on der Formel

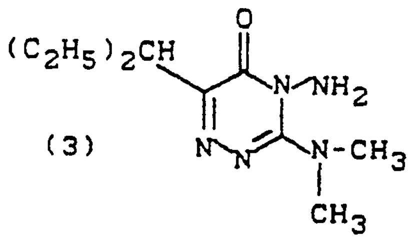

(3)

und
d) 4-Amino-3-ethylmethylamino-6-(pent-3-yl)-1,2,4-triazin-5(4H)-on der Formel

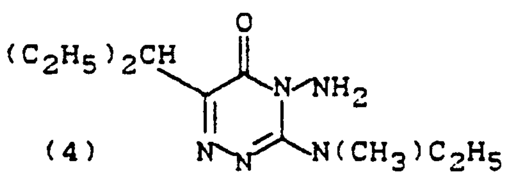

(4) ,

gemäß Anspruch 1.

5. Verfahren zur Herstellung von 6-(Pent-3-yl)-1,2,4-triazin-5(4H)-onen der Formel (I) gemäß Anspruch 1, dadurch gekennzeichnet, daß man

(a) 4-Amino-3-mercapto-6-(pent-3-yl)-1,2,4-triazin5(4H)-on der Formel (II)

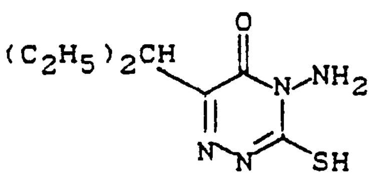

(II)

in alkalischer Lösung mit einem Alkylhalogenid, vorzugsweise Alkyliodid oder -bromid, umsetzt und man gegebenenfalls

(b) die nach Verfahren (a) erhaltenen 3-Alkylthio-4-amino-6-(pent-3-yl)-1,2,4-triazin-5(4H)-one der Formel (Ia)

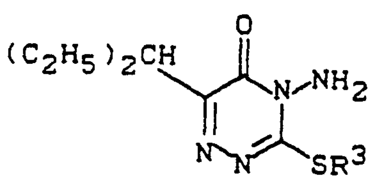

(Ia)

in welcher
$R^3$ für Alkyl, vorzugsweise mit 1 bis 4 Kohlenstoffatomen, steht,
mit einem Methylierungsmittel in Gegenwart eines Phasentransferkatalysators in einem Zweisphasensystem umsetzt, oder daß man

(c) die nach Verfahren (a) erhaltenen 3-Alkylthio-4-amino-6-(pent-3-yl)-1,2,4-triazin-5(4H)-one der Formel (Ia)

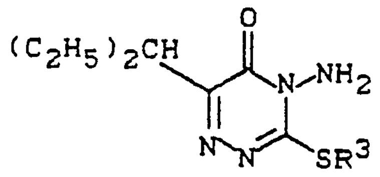

(Ia)

in welcher
$R^3$ für Alkyl, vorzugsweise mit 1 bis 4 Kohlenstoffatomen, steht,
mit Aminen der Formel (III)
$HNR^4R^5$ (III)
in welcher
$R^4$ für Wasserstoff oder Alkyl, vorzugsweise mit 1 bis 4 Kohlenstoffatomen, steht und
$R^5$ für Alkyl, vorzugsweise mit 1 bis 4 Kohlenstoffatomen, steht,
gegebenenfalls in Gegenwart eines Verdünnungsmittels und gegebenenfalls in Gegenwart einer niederen aliphatischen Carbonsäure umsetzt.

6. Herbizide und pflanzenwachstumsregulierende Mittel, gekennzeichnet durch einen Gehalt an mindestens einem 6-(Pent-3-yl)-1,2,4-triazin-5(4H)-on der Formel (I) gemäß Anspruch 1.

7. Verwendung von 6-(Pent-3-yl)-1,2,4-triazin-5(4H)-onen der Formel (I) gemäß Anspruch 1 zur Bekämpfung von unerwünschtem Pflanzenwachstum und/oder zur Regulierung des Pflanzenwachstums.

8. Verfahren zur Herstellung von herbiziden und pflanzenwachstumsregulierende Mitteln, dadurch gekennzeichnet, daß man 6-(Pent-3-yl)-1,2,4-triazin-5(4H)-one der Formel (I) gemäß Anspruch 1 mit Streckmitteln und/oder oberflächenaktiven Stoffen vermischt.

Europäisches Patentamt

# EUROPÄISCHER RECHERCHENBERICHT

Nummer der Anmeldung

EP 90 10 8892

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | EP-A-0 150 677 (CIBA-GEIGY AG)<br>* Ansprüche 11,15-20; Beispile 2.17,2.20 * | 1-4,6-8 | C 07 D 253/06<br>A 01 N 43/707 |
| Y | * Ansprüche 1,2,4-9,11,13-20; Beispiele 1.49,2.17,2.20 *<br>--- | 1-8 | |
| Y | EP-A-0 144 668 (BAYER AG)<br>* Beispiel 8 *; & DE-A-3339859 (Kat. D)<br>--- | 1-8 | |
| A | US-A-4 057 417 (K. DICKORE et al.)<br>* Beispiel 1,5,6; Ansprüche 1,2,6,10 *<br>----- | 1-8 | |
| | | | **RECHERCHIERTE SACHGEBIETE (Int. Cl.5)** |
| | | | C 07 D 253/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 22-08-1990 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)